# EUROPEAN PATENT APPLICATION

(11) **EP 4 375 661 A1**
(43) Date of publication of application: **29.05.2024**
(21) Application number: 22845900.4
(22) Date of filing: 19.07.2022
(51) Int. Cl.: G01N 33/15, G01N 33/50, G01N 33/53, A01K 67/027, C07K 14/705

(54) **Aß BIOMARKER IN ALZHEIMER'S DISEASE MODEL MOUSE AND METHOD FOR ANALYZING SAME**

(30) Priority: 21.07.2021 JP 2021120750
(71) Applicant: Shimadzu Corporation, Nakagyo-ku, Kyoto-shi, Kyoto 604-8511 (JP); The University of Tokyo, Bunkyo-ku, Tokyo 113-8654 (JP)
(72) Inventor: KANEKO, Naoki, Kyoto-shi, Kyoto 604-8511 (JP); TOMITA, Taisuke, Tokyo 113-8654 (JP); MATSUZAKI, Masaya, Tokyo 113-8654 (JP); YOKOYAMA, Miyabishara, Tokyo 113-8654 (JP)
(74) Representative: Müller-Boré & Partner Patentanwälte PartG mbB
(86) International application number: PCT/JP2022/027975
(87) International publication number: WO 2023/002967

(57) **Abstract**

A level of mouse Aβ1-40 and a level of mouse APP669-711 in a biological sample derived from an AD model mouse are measured by detection of markers including mouse Aβ1-40 and mouse APP669-711; an APP669-711/Aβ1-40 ratio which is a ratio of the level of mouse APP669-711 to the level of mouse Aβ1-40 is calculated; and when the ratio in the AD model mouse is higher than the same ratio in a reference mouse in which cerebral Aβ deposition is absent, it is judged that an amount of cerebral Aβ deposition in the AD model mouse is higher than an amount of cerebral Aβ deposition in the reference mouse.

## Description

### TECHNICAL FIELD

The present invention relates to an Aβ biomarker in a model mouse of Alzheimer's disease (AD) and an analysis method thereof.

### BACKGROUND ART

AD is a neurodegenerative disease that serves as a primary cause of dementia and accounts for at least half the entire dementia population. The number of dementia patients worldwide is estimated at about 50 millions in 2017, and expected to increase to reach about 82 millions in 2030. In Japan, the number of dementia patients is estimated to reach 7 millions in 2025.

It is believed that the onset of AD is deeply involved with amyloid β (Aβ). Amyloid precursor protein (APP), which is made up of 770 amino acid residues, is cleaved by various proteases to produce a wide range of Aβ species. Key Aβs are Aβ1-40 made up of 40 amino acid residues and Aβ1-42 made up of 42 amino acid residues, of which Aβ1-42 has a stronger tendency to aggregate. In AD brains, Aβ undergoes fibrosis and aggregation to form senile plaques, which is believed to be the earliest pathological change.

It is known that cerebral Aβ deposition starts 20 years before the onset of AD, so it is important to discriminate the possibility of AD onset earlier. As diagnostic test methods for AD, there are PET (Positron Emission Tomography) imaging that allows for visualization of cerebral Aβ deposition (amyloid PET), as well as a method that allows for detection of Aβ in cerebrospinal fluid (CSF) as a biomarker (which is also simply called "a marker"). Among these, the amyloid PET technique allows for high-accuracy imaging of the site of cerebral Aβ amyloid deposition and its amount, and has now established a position as an imaging biomarker for detecting early-stage pathological conditions of AD. However, the amyloid PET testing is costly, so, realistically, in clinical settings, it should be used only under limited conditions. The CSF testing, on the other hand, is highly invasive and puts a heavy burden on the body. In such circumstances, development of a biomarker that can be collected easily in a non-invasive manner has been demanded.

In recent years, a promising biomarker capable of reflecting the state of cerebral Aβ deposition has been suggested; reports have suggested that IP-MS (Immunoprecipitation-Mass Spectrometry) (PTL 1) (which is a combination of immuno precipitation (IP) with MALDI-TOF MS (Matrix Assisted Laser Desorption/Ionization-Time of Flight Mass Spectrometry)) can be conducted on human plasma samples, and the resulting ratios APP669-711/Aβ1-42 and Aβ1-40/Aβ1-42 in plasma as well as their composite (a composite biomarker) can give accurate prediction of cerebral Aβ deposition in humans (PTLs 2, 3, NPLs 1, 2). These biomarkers hold a potential in determination of human AD as well as for use as a risk factor for AD onset, and therefore are expected to play an important role in determination of the intervention method such as treatment and prevention, promising its clinical utility.

There is no established methods for fundamental treatment or onset prevention of AD, so development thereof is urgently needed. For research and development of intervention methods and for identifying the mechanism of AD onset, experiments based on AD model mice will be needed; then, at the time of implementing those experiments, in order for grasping when cerebral Aβ deposition starts in AD model mice and how it proceeds, for determining the timing of intervention, and for evaluating the efficacy of the intervention method at investigative stages, biomarkers will be useful, so biomarkers have potential in fundamental researches and non-clinical tests conducted by pharmaceutical companies and research institutions.

It is becoming evident that not only Aβ1-42 but also Aβ1-40 (Aβ40) are deposited in the brains of AD model mice. For example, in NPL 3, staining with Aβ1-40-specific antibody BA27 indicates deposition of Aβ1-40 in the brains of AD model mice, and NPL 4 suggests that Aβ1-40 levels in mouse cerebral FA soluble Ab (a fraction in which Aβ deposition is dissolved) are higher in transgenic mice than in wild-type (WT) mice.

### CITATION LIST

### PATENT LITERATURE

PTL 1: Japanese Patent No. 6424757
PTL 2: Japanese Patent No. 6410810
PTL 3: Japanese Patent No. 6467512

### NON PATENT LITERATURE

NPL 1: Kaneko N, Nakamura A, Washimi Y, Kato T, Sakurai T, Arahata Y, Bundo M, Takeda A, Niida S, Ito K, Toba K, Tanaka K, Yanagisawa K.: Novel plasma biomarker surrogating cerebral amyloid deposition. Proc Jpn Acad Ser B Phys Biol Sci. 2014;90 (9):353-64
NPL 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K.: High performance plasma amyloid-β biomarkers for Alzheimer's disease. Nature. 2018;8;554 (7691):249-254
NPL 3: Antibody catalogue from Fujifilm Wako "For Alzheimer's Disease Research Anti Amyloid β Antibody BAN50/BNT77/BA27/BC05" [retrieved on June 8, 2021], Internet <URL:https://labchem-wako.fujifilm.com/us/category/docs/01222_doc02.pdf>
NPL 4: Li X, Feng Y, Wu W, Zhao J, Fu C, Li Y, Ding Y, Wu B, Gong Y, Yang G, Zhou X. "Sex differences between APPswePS1dE9 mice in A-beta accumulation and pancreatic islet function during the development of Alzheimer's disease", Lab Anim. 2016 Aug;50 (4):275-85
NPL 5: Franke TN, Irwin C, Bayer TA, Brenner W, Beindorff N, Bouter C, Bouter Y.: In vivo Imaging With 18 F-FDG- and 18 F-Florbetaben-PET/MRI Detects Pathological Changes in the Brain of the Commonly Used 5XFAD model mouse of Alzheimer's Disease. Front Med (Lausanne). 2020 Sep 15;7:529
NPL 6: Loffler T, Flunkert S, Temmel M, Hutter-Paier B.: Decreased Plasma Aβ in Hyperlipidemic APPSL Transgenic Mice Is Associated with BBB Dysfunction Front Neurosci. 2016 Jun 1;10:232
NPL 7: Wu Q, Li Q, Zhang X, Ntim M, Wu X, Li M, Wang L, Zhao J, Li S.: Treatment with Bifidobacteria can suppress Aβ accumulation and neuroinflammation in APP/PS1 mice. PeerJ. 2020 Oct 28;8:e10262
NPL 8: "Research Models" [retrieved on June 14, 2021], Internet <https://www.alzforum.org/research-models/alzheimers-disease>
NPL 9: "B6.Cg-Tg(APPswe,PSEN1dE9)85Dbo/Mmjax" [retrieved on June 14, 2021], Internet <https://www.jax.org/strain/005864>
NPL 10: "B6.Cg-Tg(APPSwFlLon,PSEN1*M146L*L286V)6799Vas/Mmjax" [retrieved on June 14, 2021], Internet <https://www.jax.org/strain/008730>
NPL 11: "B6;C3- Tg(APPswe,PSEN1 dE9)85Dbo/Mmj ax" [retrieved on June 14, 2021], Internet <https://www.jax.org/strain/004462>
NPL 12: "APP/PS1/rTg21221" [retrieved on June 14, 2021], Internet <https://www.alzforum.org/research-models/appps1rtg21221>
NPL 13: "B6.Cg-Tg(APP695)3Dbo Tg(PSEN1dE9)S9Dbo/Mmjax" [retrieved on June 14, 2021], Internet <https://www.jax.org/strain/005866>
NPL 14: "B6;C3-Tg(APPswe,PSEN1dE9)85Dbo/Mmjax" [retrieved on June 14, 2021], Internet <https://www.jax.org/strain/004462>
NPL 15: "APPSWE-Model 1349" [retrieved on June 14, 2021], Internet <https://www.taconic.com/transgenic-mouse-model/appswe-model-1349>
NPL 16: "APPSWE-Tau" [retrieved on June 14, 2021], Internet <https://www.taconic.com/transgenic-mouse-model/appswe-tau>
NPL 17: "B6SJL-Tg(APPSwFlLon,PSEN1*M146L*L286V)6799Vas/Mmjax" [retrieved on June 14, 2021], Internet <https://www.jax.org/strain/006554>
NPL 18: "B6;129-Tg(APPSwe,tauP301L)1LfaPsen1tm1Mpm/Mmjax" [retrieved on June 14, 2021], Internet <https://www.jax.org/strain/004807>
NPL 19: "B6.Cg-Tg(Thy1-APP)3Somm/J" [retrieved on June 14, 2021], Internet <https://www.jax.org/strain/030504>
NPL 20: "STOCK Apptm1Sud/J" [retrieved on June 14, 2021], Internet <https://www.jax.org/strain/008390>
NPL 21: Macklin L, Griffith CM, Cai Y, Rose GM, Yan XX, Patrylo PR.: Glucose tolerance and insulin sensitivity are impaired in APP/PS1 transgenic mice prior to amyloid plaque pathogenesis and cognitive decline. Exp Gerontol. 2017 Feb;88:9-18

### SUMMARY OF INVENTION

### TECHNICAL PROBLEM

The above-mentioned PET imaging technique for evaluating the amount of cerebral Aβ deposition in AD model mice requires a PET apparatus designed for use with laboratory animals, and the experiments present a potential hazard because radioactive substances need to be used (NPL 5). In addition, because AD model mice are small in size, their CSF is difficult to collect and can be small in amount, also potentially accompanied by a risk of blood contamination. There is another, easier method for evaluating cerebral Aβ deposition, which involves dissolving the brains of AD model mice and then measuring amyloid in insoluble fractions to evaluate the state of cerebral amyloid deposition, but this method sacrifices the AD model mice, so continuous monitoring is impossible (NPLs 6, 7).

The present invention aims at providing a biomarker that enables evaluation of the state of cerebral Aβ deposition in AD model mice and that is adapted for relatively easy collection, safety, and continuous monitoring, as well as an analysis method thereof.

### SOLUTION TO PROBLEM

A first aspect of the present invention is directed to an analysis method of judging a state of cerebral Aβ deposition, the analysis method comprising:
measuring a level of mouse Aβ1-40 and a level of mouse APP669-711 in a biological sample derived from an AD model mouse, by detection of markers including mouse Aβ1-40 and mouse APP669-711;
calculating an APP669-711/Aβ1-40 ratio which is a ratio of the level of mouse APP669-711 to the level of mouse Aβ1-40; and
when the ratio in the AD model mouse is higher than the same ratio in a reference mouse in which cerebral Aβ deposition is absent, judging that an amount of cerebral Aβ deposition in the AD model mouse is higher than an amount of cerebral Aβ deposition in the reference mouse.

A second aspect of the present invention is directed to an analysis method of judging efficacy of an intervention performed on an AD model mouse, in terms of a state of cerebral Aβ deposition, the analysis method comprising:
before and after the intervention performed on the AD model mouse,
   measuring a level of mouse Aβ1-40 and a level of mouse APP669-711 in a biological sample derived from the AD model mouse, by detection of markers including mouse Aβ1-40 and mouse APP669-711, and
   calculating an APP669-71 1/Aβ1-40 ratio which is a ratio of the level of mouse APP669-711 to the level of mouse Aβ1-40; and
comparing the ratio in the AD model mouse before the intervention with the ratio in the AD model mouse after the intervention.

A third aspect of the present invention is directed to an analysis method of judging whether an amount of cerebral Aβ deposition is increased or decreased, the analysis method comprising:
performing, on a same AD model mouse over a period of time, multiple sets of a step of measuring a level of mouse Aβ1-40 and a level of mouse APP669-711 in a biological sample derived from the AD model mouse by detection of markers including mouse Aβ1-40 and mouse APP669-711 and a step of calculating an APP669-711/Aβ1-40 ratio which is a ratio of the level of mouse APP669-711 to the level of mouse Aβ1-40; and
evaluating a change in the ratio in the AD model mouse.

A fourth aspect of the present invention is directed to an analysis method of screening candidate substances intended for improving a state of cerebral Aβ deposition, the analysis method comprising:
before and after providing an AD model mouse with a candidate substance intended for improving a state of cerebral Aβ deposition,
   measuring a level of mouse Aβ1-40 and a level of mouse APP669-711 in a biological sample derived from the AD model mouse, by detection of markers including mouse Aβ1-40 and mouse APP669-711, and
   calculating an APP669-71 1/Aβ1-40 ratio which is a ratio of the level of mouse APP669-711 to the level of mouse Aβ1-40; and
comparing the ratio in the AD model mouse before the candidate substance is provided, with the ratio in the AD model mouse after the candidate substance is provided.

A fifth aspect of the present invention is directed to a marker enabling judgement of a state of cerebral Aβ deposition and consisting of a combination of mouse Aβ1-40 and mouse APP669-711 in a biological sample derived from an AD model mouse.

### ADVANTAGEOUS EFFECTS OF INVENTION

With the analysis method and the marker according to the present invention, it is possible to provide an analysis method and a biomarker that enable evaluation of the state of cerebral Aβ deposition in AD model mice and that are adapted for relatively easy collection, safety, and continuous monitoring.

### BRIEF DESCRIPTION OF DRAWINGS

Fig. 1 is an immunostaining image of brain tissue of a 23-month-old APP/PS1 mouse in Example 1.
Fig. 2 shows mass spectra of mouse Aβ-related peptides in plasma of APP/PS1 mice of 2-month-old (A) and of 25-month-old (B) in Example 1.
Fig. 3 is a graph comparing the normalized intensity of plasma Aβ1-40 in young AD model mice (2- to 5-month-old, N=11) and the normalized intensity of plasma Aβ1-40 in old AD model mice (23- to 30-month-old, N=13) in Example 1.
Fig. 4 is a graph comparing the normalized intensity of plasma APP669-711 in young AD model mice (2- to 5-month-old, N=11) and the normalized intensity of plasma APP669-711 in old AD model mice (23- to 30-month-old, N=13) in Example 1.
Fig. 5 is a graph comparing the plasma APP669-711/Aβ1-40 ratio in young AD model mice (2- to 5-month-old, N=11) and the plasma APP669-711/Aβ1-40 ratio in old AD model mice (23- to 30-month-old, N=13) in Example 1.

### DESCRIPTION OF EMBODIMENTS

### <Analysis Method>

### (First Aspect)

A first aspect of the present invention is an analysis method of judging a state of cerebral Aβ deposition, the analysis method comprising:
measuring a level of mouse Aβ1-40 and a level of mouse APP669-711 in a biological sample derived from an AD model mouse, by detection of markers including mouse Aβ1-40 and mouse APP669-711;
calculating an APP669-711/Aβ1-40 ratio which is a ratio of the level of mouse APP669-711 to the level of mouse Aβ1-40; and
when the ratio in the AD model mouse is higher than the same ratio in a reference mouse in which cerebral Aβ deposition is absent, judging that an amount of cerebral Aβ deposition in the AD model mouse is higher than an amount of cerebral Aβ deposition in the reference mouse.

The first aspect includes a step of measuring a level of mouse Aβ1-40 and a level of mouse APP669-711 in a biological sample derived from an AD model mouse, by detection of markers including mouse Aβ1-40 and mouse APP669-711 (this step is referred to as "a measurement step"). The AD model mouse is not particularly limited, and, for example, the AD model mice described in NPL 8 and the like can be used as appropriate. Specific examples thereof include
Tg(APPswe,PSEN1dE9)85Dbo (NPL 9), 5xFAD(B6SJL) (NPL 10), 5xFAD(C57BL6) (NPL 11), APP/PS1/rTg21221 (NPL 12), APPSwe/PSEN1(A246E) (NPL 13), APPSwe/PSEN1dE9 (C3-3 x S-9) (NPL 14), APPswe/PSEN1dE9 (line 85) (NPL 15), Tg2576/Tau(P301L)(APPSwe-Tau) (NPL 16), TREM2-BAC X 5xFAD (NPL 17), 3xTg (NPL 18), APP23 (NPL 19), and APP Knock-in (NPL 20).

As the AD model mouse, an APP/PS1 mouse which is a transgenic mouse with overexpression of human Swedish mutant APP and ΔExon mutant PS1 driven by nervous-system-specific prion promoter (PrP) is preferable, and, in Example 1 below, Tg(APPswe,PSEN1dE9)85Dbo is used. APP/PS1 mice are known to develop cerebral Aβ deposition starting at about 6 to 7 months old (NPL 21), and, therefore, young and old APP/PS1 mice are used when investigating the presence or absence of cerebral Aβ deposition.

The "biological sample" in the measurement step can be selected from blood, urine, stool, biological secretory fluid (such as saliva, lacrimal fluid, for example), and the like. Among these, blood is preferable as the biological sample because it is easy to collect and highly reflective of the state of cerebral Aβ. The blood sample is directly subjected to the step of marker expression level measurement, and includes whole blood, plasma, serum, and the like. The blood can be prepared by properly treating whole blood collected from an AD model mouse. The treatment to perform for preparation of a blood sample from whole blood thus collected is not particularly limited, and, for example, any treatment such as centrifugation and/or the like may be performed. The blood for use in the measurement step may have undergone low-temperature storage such as freezing, as appropriate, during the preparation thereof or after the preparation thereof.

The biological sample may be used as it is for measurement of the concentrations of its components, or may be used for measurement of the concentrations of its components after proper pretreatment, as needed. Examples of the pretreatment include termination of enzymatic reaction in the biological sample, removal of fat-soluble substances, removal of proteins, and the like. The pretreatment may be carried out by a known method. Also, the biological sample may be used after dilution or concentration, as appropriate.

In the measurement step, the level of mouse Aβ1-40 and the level of mouse APP669-711 in the biological sample are measured. Mouse Aβ1-40 and mouse APP669-711 have the following amino acid sequences, respectively.

### Mouse Aβ1-40:

DAEFGHDSGFEVRHQKLVFFAEDVGSNKGAIIGLMVGGVV (SEQ ID NO: 1)
Mouse APP699-711:
VKMDAEFGHDSGFEVRHQKLVFFAEDVGSNKGAIIGLMVGGVV (SEQ ID NO: 2)

In the present invention, the level of a certain marker basically means the concentration thereof; however, it may also be another unit that a person skilled in the art would use instead of concentrations, and, for example, it may be ionic strength detected by mass spectrometry.

Preferably, measurement of markers is carried out by a test based on biomolecule specific affinity. The test based on biomolecule specific affinity is a method well-known to those skilled in the art and is not particularly limited, but preferably it is an immunoassay. Specific examples thereof include immunoassays including competitive and non-competitive immunoassays, such as Western blotting, radioimmunoassay, ELISA (Enzyme-Linked ImmunoSorbent Assay) (including sandwich immunoassay, competitive assay, and direct adsorption assay), immuno precipitation assay, sedimentation reaction, immunodiffusion assay, immunoaggregation assay, complement binding reaction analysis, immunoradiometric assay, fluorescence immunoassay, and protein A immunoassay. In the immunoassay, an antibody that binds to the marker in the biological sample is detected.

Measurement of markers may also be carried out by using an immunoglobulin that has an antigen-binding site capable of recognizing a peptide derived from amyloid precursor protein (APP), or an antibody-immobilized support that is prepared with the use of an immunoglobulin fragment containing an antigen-binding site capable of recognizing a peptide derived from amyloid precursor protein (APP). By immuno precipitation assay using the above-mentioned antibody-immobilized support, peptides in a sample can be detected on a mass spectrometer (IP-MS).

The first aspect includes, after the above-described measurement step, a step of calculating an APP669-711/Aβ1-40 ratio which is a ratio of the level of mouse APP669-711 to the level of mouse Aβ1-40 (this step is referred to as "a calculation step"). This ratio APP669-711/Aβ1-40, namely a marker, is observed to be significantly different between the ratio in a plasma sample from a reference AD model mouse in which cerebral Aβ deposition is absent and the ratio in a plasma sample from an AD model mouse in which cerebral Aβ deposition is excessive.

Conventionally, in analysis of AD in humans and the like, Aβ1-42 has been used as a preferable indicator (PTLs 2, 3, NPLs 1, 2). Similarly in mice, there exists mouse Aβ1-42 which has the following amino acid sequence.

Mouse Aβ1-42:
DAEFGHDSGFEVRHQKLVFFAEDVGSNKGAIIGLMVGGVVIA (SEQ ID NO: 3)

It should be noted that when using AD model mice, unlike in humans and the like, the amount of blood to be collected is small, so the mouse Aβ1-42 the concentration of which is low is difficult to measure. In that context, the inventors of the present invention have found that when using AD model mice, mouse Aβ1-40 and mouse APP669-711 may be used as more suitable indicators than mouse Aβ1-42.

The first aspect includes, after the above-described calculation step, a step of judging that, when the ratio in the AD model mouse is higher than the same ratio in a reference mouse in which cerebral Aβ deposition is absent, an amount of cerebral Aβ deposition in the AD model mouse is higher than an amount of cerebral Aβ deposition in the reference mouse. In this step, the measured value is compared to the reference value to analyze the marker concentration in the biological sample. As the reference mouse, an AD model mouse of 2- to 5-month-old (young) and a wild-type mouse can be preferably used.

With this analysis method according to the first aspect, it is possible to provide an analysis method that enables evaluation of the state of cerebral Aβ deposition in AD model mice and that is adapted for relatively easy collection, safety, and continuous monitoring.

### (Second Aspect)

A second aspect of the present invention is an analysis method of judging efficacy of an intervention performed on an AD model mouse, in terms of a state of cerebral Aβ deposition, the analysis method comprising:
before and after the intervention performed on the AD model mouse,
   measuring a level of mouse Aβ1-40 and a level of mouse APP669-711 in a biological sample derived from the AD model mouse, by detection of markers including mouse Aβ1-40 and mouse APP669-711 (a measurement step), and
   calculating an APP669-71 1/Aβ1-40 ratio which is a ratio of the level of mouse APP669-711 to the level of mouse Aβ1-40 (a calculation step); and
comparing the ratio in the AD model mouse before the intervention with the ratio in the AD model mouse after the intervention.

The analysis method according to the second aspect is implemented before and after the intervention performed on the AD model mouse. Herein, "intervention" includes administration of therapeutic agents and/or preventive agents for AD, diet therapy, exercise therapy, learning therapy, surgery, and the like. Each of the exercise therapy, learning therapy, and surgery includes both known and unknown ones.

The measurement step and the calculation step according to the second aspect are the same as the measurement step and the calculation step according to the first aspect described above.

In the second aspect, following the measurement step and the calculation step implemented before and after the intervention performed on the AD model mouse, comparison of the ratio in the AD model mouse before the intervention with the ratio in the AD model mouse after the intervention is carried out. With this second aspect thus applied before and after the intervention performed on the AD model mouse, it is possible to evaluate the drug efficacy of therapeutic agents and/or preventive agents for AD or to evaluate the efficacy of other treatments.

### (Third Aspect)

A third aspect of the present invention is an analysis method of judging whether an amount of cerebral Aβ deposition is increased or decreased, the analysis method comprising:
performing, on a same AD model mouse over a period of time, multiple sets of a step of measuring a level of mouse Aβ1-40 and a level of mouse APP669-711 in a biological sample derived from the AD model mouse by detection of markers including mouse Aβ1-40 and mouse APP669-711 (a measurement method) and a step of calculating an APP669-711/Aβ1-40 ratio which is a ratio of the level of mouse APP669-711 to the level of mouse Aβ1-40 (a calculation step); and
evaluating a change in the ratio in the AD model mouse.

The analysis method according to the third aspect performs multiple sets of the measurement step and the calculation step on the same AD model mouse over a period of time. The measurement step and the calculation step according to the third aspect are the same as the measurement step and the calculation step according to the first aspect described above.

In the third aspect, multiple sets of the measurement step and the calculation step are performed on the same AD model mouse over a period of time and a change in the ratio in the AD model mouse is evaluated. With this third aspect, it is possible to evaluate over-time changes in cerebral Aβ deposition in an AD model mouse and judge whether the amount of cerebral Aβ deposition is increased or decreased.

### (Fourth Aspect)

A fourth aspect of the present invention is an analysis method of screening candidate substances intended for improving a state of cerebral Aβ deposition, the analysis method comprising:
before and after providing an AD model mouse with a candidate substance intended for improving a state of cerebral Aβ deposition,
   measuring a level of mouse Aβ1-40 and a level of mouse APP669-711 in a biological sample derived from the AD model mouse, by detection of markers including mouse Aβ1-40 and mouse APP669-711 (a measurement step), and
   calculating an APP669-71 1/Aβ1-40 ratio which is a ratio of the level of mouse APP669-711 to the level of mouse Aβ1-40 (a calculation step); and
comparing the ratio in the AD model mouse before the candidate substance is provided, with the ratio in the AD model mouse after the candidate substance is provided.

The analysis method according to the fourth aspect is implemented before and after providing an AD model mouse with a candidate substance intended for improving the state of cerebral Aβ deposition. Herein, the "candidate substance" refers to an unknown substance that holds a potential as a candidate of a therapeutic agent and/or a preventive agent for AD usable for improving the state of cerebral Aβ deposition.

The measurement step and the calculation step according to the fourth aspect are the same as the measurement step and the calculation step according to the first aspect described above.

In the fourth aspect, comparison of the ratio in the AD model mouse before the candidate substance is provided with the ratio in the AD model mouse after the candidate substance is provided is carried out. With this fourth aspect thus applied before and after providing an AD model mouse with a candidate substance intended for improving the state of cerebral Aβ deposition, it is possible to evaluate the efficacy of the candidate substance intended for improving the state of cerebral Aβ deposition.

### <Marker Enabling Judgement of State of Cerebral Aβ Deposition>

### (Fifth Aspect)

The present invention also provides a marker enabling judgement of a state of cerebral Aβ deposition and consisting of a combination of mouse Aβ1-40 and mouse APP669-711 in a biological sample derived from an AD model mouse. This can be regarded as a biomarker specific for AD model mice, because the blood APP669-711/Aβ1-40 ratio in humans has not been reported as a biomarker. With the use of the marker according to the present invention, it is possible to accurately judge the state of cerebral Aβ deposition in a biological sample derived from an AD model mouse.

The biological sample and the AD model mouse in the context of the marker according to the present invention may be the same as those described above for the first aspect; similarly, the biological sample is preferably blood, and the AD model mouse is preferably an APP/PS1 mouse.

In the following, a more detailed description will be given of the present invention with reference to examples; however, it should be noted that the below description does not limit the present invention.

### <Example 1>

### [1] AD Model Mouse

An APP/PS1 mouse (Tg(APPswe,PSEN1dE9)85Dbo, NPL 9), which was a widely-used AD model mouse, was used. An APP/PS1 mouse is a transgenic mouse with overexpression of human Swedish mutant APP and ΔExon mutant PS1 driven by nervous-system-specific prion promoter (PrP). APP/PS1 mice of 2- to 5-month-old (young) and those of 23- to 30-month-old (old) were used. APP/PS1 mice are known to develop cerebral Aβ deposition starting at about 6 to 7 months old (NPL 21), and, therefore, young and old APP/PS1 mice are used when investigating the presence or absence of cerebral Aβ deposition.

### [2] Immunostaining of Brain Tissue

In a 23-month-old APP/PS1 mouse used in the present example, by immunostaining of the brain tissue, development of cerebral Aβ deposition was confirmed in the procedure described below. A half portion of the brain of the mouse was resected and impregnated with 4% PFA (paraformaldehyde)/PBS (Phosphate Buffer Solution) (pH6.7) for 24 hours, and the brain was cut into narrow pieces. Subsequently, the brain was dehydrated and embedded with the use of 70% ethanol for 1 hour, 80% ethanol for 2 hours, 90% ethanol for 2 hours, 99% ethanol for 3 hours (×3 times), xylene for 3 hours, xylene for 2 hours, and paraffin for 3 hours (×3 times). The resulting block was sliced into a thickness of 4 mm with MICROM HM 430 (Thermo SCIENTIFIC), spread on a slide glass coated with PLL (poly-L-lysine), and dried at 37°C for 2 days.

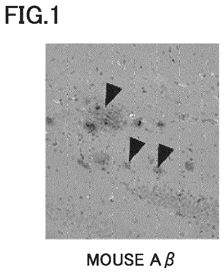

For deparaffinization, the brain slice was soaked in xylene for 5 minutes, and this was repeated 3 times. Then, it was soaked in 100% ethanol for 2.5 minutes (×2 times), 90% ethanol for 2.5 minutes, 80% ethanol for 2.5 minutes, 70% ethanol for 2.5 minutes, and running water for 10 minutes. For antigen activation, autoclaving was carried out in 0.01-M citric acid (pH6.0)/DW (distilled water) at 121°C for 10 minutes, followed by soaking in running water for 10 minutes and in TS (Tris buffer). Subsequently, the sample was subjected to blocking with inactivated 10% calf serum/PBS at room temperature for 30 minutes, and made coexist overnight with various 1000-fold-diluted primary antibodies at room temperature. As the primary antibodies, anti-human Aβ mouse monoclonal antibody (82E1, IBL) and anti-mouse Aβ rabbit polyclonal antibody (APP597, IBL) were used. Then, the specimen was soaked with various 500-fold-diluted biotinylated secondary antibodies at room temperature for 2 hours, ABC elite (VECTOR) for 1 hour, ImmPACT SG (VECTOR) for 5 minutes, 0.1% hematoxylin liquid for 10 seconds, and running water for 5 minutes. Lastly, for dehydration, the specimen was soaked in 70% ethanol for 2.5 minutes, 80% ethanol for 2.5 minutes, 90% ethanol for 2.5 minutes, 100% ethanol for 2.5 minutes (×2 times), and for clearing, xylene for 5 minutes (×3 times). Then, the specimen was sealed with a sealing agent, HSR liquid (Sysmex), dried, and then examined with EVOS FL Auto2 (Invitrogen); in this manner, in a 23-month-old APP/PS1 mouse, by immunostaining of the brain tissue, development of cerebral Aβ deposition was confirmed (Fig. 1).

### [3] Blood Collection from AD Model Mouse

A 21G needle was put into a vein of an AD model mouse, in the face near the jaw bone, and the blood oozed out to the surface was collected. For preventing coagulation of the blood during the blood collection, EDTA-2Na (final concentration, 0.15%) was added in advance to respective blood collection tubes. The blood thus collected was immediately centrifuged at 4°C and 3,500 rpm for 5 minutes, and the supernatant, which was plasma, was collected. Each blood component thus obtained was cryopreserved at -80°C.

### [4] Measurement of Plasma Aβ-related Peptides

Aβ-related peptides in the plasma were measured by IP-MS, which is a combination of immunoprecipitation (IP) and mass spectrometry (MS). Anti-mouse Aβ rabbit polyclonal antibody APP597 (IBL) was purified with the use of Protein G (Thermo Fisher Scientific) for removal of BSA, and then the antibody was bound to Dynabeads Epoxy (Thermo Fisher Scientific) via covalent bonding to obtain antibody beads for use in IP. The cryopreserved mouse plasma (40 to 100 µL) was thawed, and the plasma was mixed with an equal amount of an internal standard peptide solution. The resultant was mixed with the antibody beads, and then subjected to antigen-antibody reaction for 1 hour at 4°C. As the internal standard peptide, 40-pM stable isotope labeled mouse Aβ1-42 (SIL-Aβ1-42, with a theoretical mass m/z of 4471.30) was used. After the antigen-antibody reaction, the antibody beads were washed, followed by elution of Aβ-related peptides with a glycine buffer (pH2.8) containing DDM (n-dodecyl-β-D-maltoside). After the resultant was brought to neutral with the use of Tris buffer, the antibody beads and the Aβ-related peptides were subjected to another round of antigen-antibody reaction, washed, followed by elution of Aβ-related peptides with the use of an elution liquid (5 mM HCl, 0.1 mM Methionine, 70% (v/v) acetonitrile). The elution liquid resulting from IP was added dropwise to four wells of a µFocus MALDI plate (trademark) 900 µm (Hudson Surface Technology, Inc., Fort Lee, NJ), which was in advance subjected to dropwise addition of 0.5 µL of 0.5 mg/mL CHCA (α-cyano-4-hydroxycinnamic acid)/0.2% (w/v) MDPNA (methylenediphosphonic acid) and drying.

Mass spectrum data was acquired with the use of AXIMA Performance (Shimadzu/KRATOS, Manchester, UK), by Linear TOF on positive ion mode. The Linear TOF m/z values were displayed as average mass of the peaks. Calibration of the m/z values and the signal intensity were carried out with the use of the standard peptides. Calibration of the m/z values was carried out with the use of human angiotensin II as well as human ACTH fragment 18-39, bovine insulin oxidized beta-chain, bovine insulin as external standards. The internal standard peptide SIL-Aβ1-42 was used for normalization of the signal intensities of Aβ-related peptides in the plasma. Subsequently, the normalized intensities of four mass spectra obtained from one sample were averaged, which was regarded as the amount of Aβ-related peptides for evaluation. In the case where there were two or more data that did not reach the lower limit of detection (S/N<3), it was judged that detection was impossible to perform.

In this measurement method, an antibody capable of specifically reacting with the sequence of mouse Aβ was used, and, in addition, as for the mass spectra, peaks that were detected at the theoretical mass of the mouse Aβ sequence were analyzed. Hence, the analysis data is about mouse Aβ-related peptides, and not about Aβ-related peptides derived from overexpressed human Swedish mutant APP.

### [5] Analysis of Plasma Aβ-related Peptides

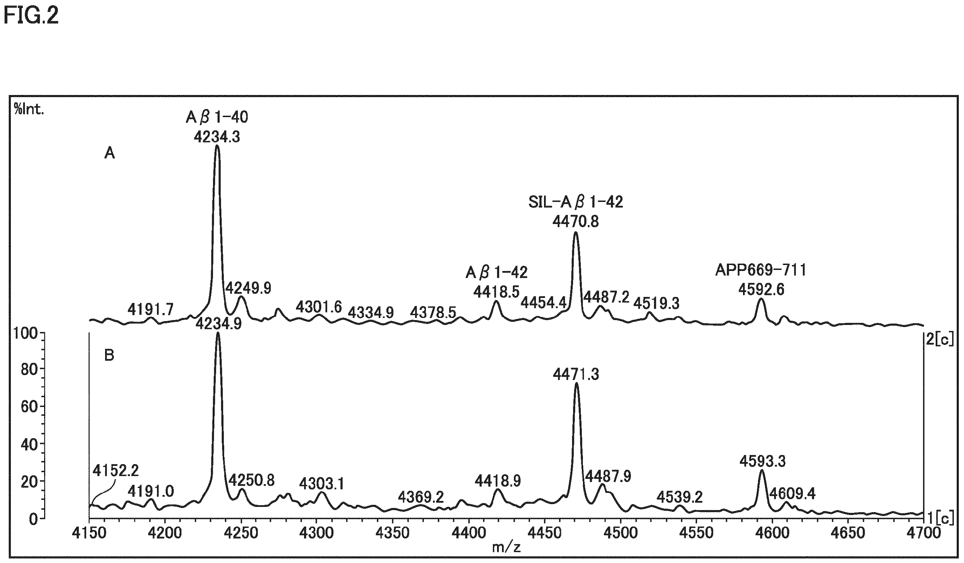

Mass spectra of mouse Aβ-related peptides obtained by IP-MS analysis of the plasma of 2-month-old and 25-month-old APP/PS1 mice are shown in Fig. 2. This data is of a single 2-month-old mouse and a single 25-month-old mouse, and in the mass spectra, the peak of each of Aβ1-40, Aβ1-42, and APP669-711 was detected at the position of the theoretical mass given in Table 1.

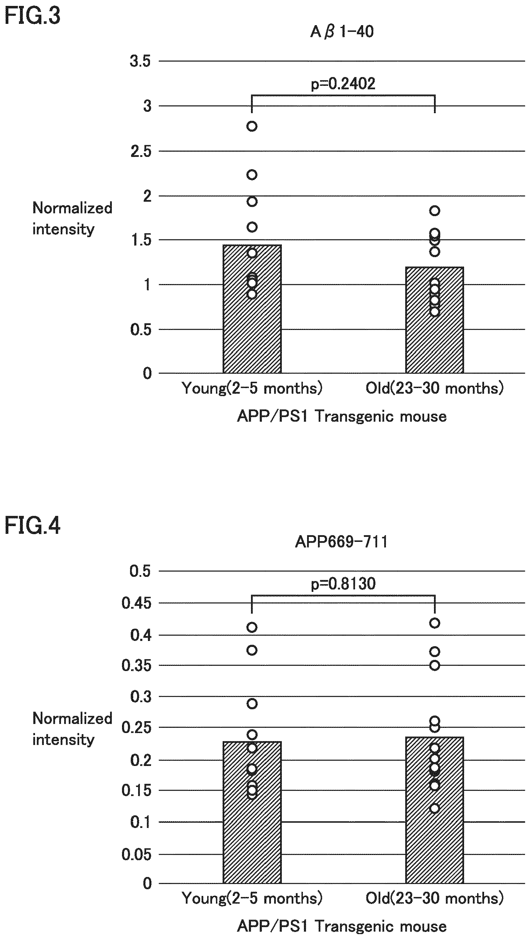

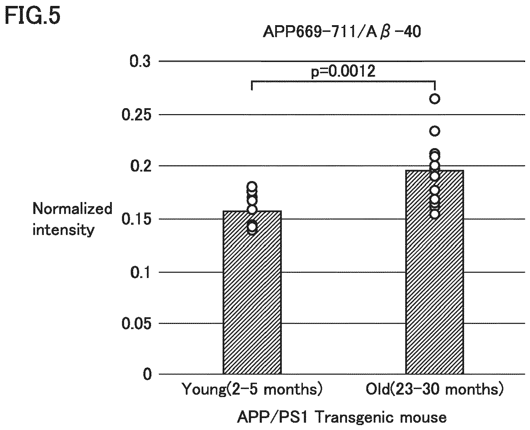

Then, categorization was made into a young group (2- to 5-month-old) without cerebral Aβ deposition and an old group (23- to 30-month-old) with cerebral Aβ deposition, and the normalized intensity of Aβ1-40 and APP669-711 was compared between these groups. The comparison between these two groups was carried out by student's t-test, with the level of significance defined at 5%. As a result, as for Aβ1-40 and APP669-711, no significant difference was found between the young group and the old group (Figs. 3, 4). On the other hand, when the ratio of the normalized intensity of APP669-711 to that of Aβ1-40 (APP669-711/Aβ1-40) was calculated and compared between the two groups, a significant difference was observed between the young group and the old group, with p=0.0012. This means that APP669-711/Aβ1-40 of the old group was higher than that of the young group (Fig. 5). From this, it was suggested that the blood APP669-711/Aβ1-40 ratio reflects cerebral Aβ deposition in an AD model mouse. This can be regarded as a biomarker specific for AD model mice, because the blood APP669-711/Aβ1-40 ratio in humans has not been reported as a biomarker.

### [Aspects]

As will be appreciated by those skilled in the art, the above-described example embodiments and experiment example(s) are specific examples of the below aspects.

### (Item 1)

An analysis method according to an aspect is an analysis method of judging a state of cerebral Aβ deposition, the analysis method comprising:
measuring a level of mouse Aβ1-40 and a level of mouse APP669-711 in a biological sample derived from an AD model mouse, by detection of markers including mouse Aβ1-40 and mouse APP669-711;
calculating an APP669-711/Aβ1-40 ratio which is a ratio of the level of mouse APP669-711 to the level of mouse Aβ1-40; and
when the ratio in the AD model mouse is higher than the same ratio in a reference mouse in which cerebral Aβ deposition is absent, judging that an amount of cerebral Aβ deposition in the AD model mouse is higher than an amount of cerebral Aβ deposition in the reference mouse.

With the analysis method according to Item 1, it is possible to provide an analysis method that enables evaluation of the state of cerebral Aβ deposition in AD model mice and that is adapted for relatively easy collection, safety, and continuous monitoring.

### (Item 2)

An analysis method according to an aspect is an analysis method of judging efficacy of an intervention, in terms of a state of cerebral Aβ deposition, the analysis method comprising:
before and after the intervention performed on an AD model mouse,
   measuring a level of mouse Aβ1-40 and a level of mouse APP669-711 in a biological sample derived from the AD model mouse, by detection of markers including mouse Aβ1-40 and mouse APP669-711, and
   calculating an APP669-71 1/Aβ1-40 ratio which is a ratio of the level of mouse APP669-711 to the level of mouse Aβ1-40; and
comparing the ratio in the AD model mouse before the intervention with the ratio in the AD model mouse after the intervention.

With the analysis method according to Item 2 thus applied before and after the intervention performed on the AD model mouse, it is possible to evaluate the drug efficacy of therapeutic agents and/or preventive agents for AD or to evaluate the efficacy of other treatments.

### (Item 3)

An analysis method according to an aspect is an analysis method of judging whether an amount of cerebral Aβ deposition is increased or decreased, the analysis method comprising:
performing, on a same AD model mouse over a period of time, multiple sets of a step of measuring a level of mouse Aβ1-40 and a level of mouse APP669-711 in a biological sample derived from the AD model mouse by detection of markers including mouse Aβ1-40 and mouse APP669-711 and a step of calculating an APP669-711/Aβ1-40 ratio which is a ratio of the level of mouse APP669-711 to the level of mouse Aβ1-40; and
evaluating a change in the ratio in the AD model mouse.

With the analysis method according to Item 3, it is possible to evaluate over-time changes in cerebral Aβ deposition in an AD model mouse and judge whether the amount of cerebral Aβ deposition is increased or decreased.

### (Item 4)

An analysis method according to an aspect is an analysis method of screening candidate substances intended for improving a state of cerebral Aβ deposition, the analysis method comprising:
before and after providing an AD model mouse with a candidate substance intended for improving a state of cerebral Aβ deposition,
   measuring a level of mouse Aβ1-40 and a level of mouse APP669-711 in a biological sample derived from an AD model mouse, by detection of markers including mouse Aβ1-40 and mouse APP669-711, and
   calculating an APP669-71 1/Aβ1-40 ratio which is a ratio of the level of mouse APP669-711 to the level of mouse Aβ1-40; and
comparing the ratio in the AD model mouse before the candidate substance is provided, with the ratio in the AD model mouse after the candidate substance is provided.

With the analysis method according to Item 4 thus applied before and after providing an AD model mouse with a candidate substance intended for improving the state of cerebral Aβ deposition, it is possible to evaluate the efficacy of a candidate substance intended for improving the state of cerebral Aβ deposition.

### (Item 5)

In the analysis method according to any one of Item 1 to Item 4, the biological sample is blood.

With the analysis method according to Item 5, collection is easy as compared to a biological sample other than blood.

### (Item 6)

In the analysis method according to any one of Item 1 to Item 5, the AD model mouse is an APP/PS1 mouse.

With the analysis method according to Item 6, the mouse is a widely-used AD model mouse and readily available.

### (Item 7)

A marker enabling judgement of a state of cerebral Aβ deposition according to an aspect consists of a combination of mouse Aβ1-40 and mouse APP669-711 in a biological sample derived from an AD model mouse.

With the marker according to Item 7, it is possible to accurately judge the state of cerebral Aβ deposition in a biological sample derived from an AD model mouse.

### (Item 8)

In the marker according to Item 7, the biological sample is blood.

With the marker according to Item 8, collection is easy as compared to a biological sample other than blood.

### (Item 9)

In the marker according to Item 7 or Item 8, the AD model mouse is an APP/PS1 mouse.

With the marker according to Item 9, the mouse is a widely-used AD model mouse and readily available.

[Sequence Listing]

## Claims

1. An analysis method of judging a state of cerebral Aβ deposition, the analysis method comprising:
measuring a level of mouse Aβ1-40 and a level of mouse APP669-711 in a biological sample derived from an Alzheimer's disease (AD) model mouse, by detection of markers including mouse Aβ1-40 and mouse APP669-711;
calculating an APP669-711/Aβ1-40 ratio which is a ratio of the level of mouse APP669-711 to the level of mouse Aβ1-40; and
when the ratio in the AD model mouse is higher than the same ratio in a reference mouse in which cerebral Aβ deposition is absent, judging that an amount of cerebral Aβ deposition in the AD model mouse is higher than an amount of cerebral Aβ deposition in the reference mouse.

2. An analysis method of judging efficacy of an intervention performed on an AD model mouse, in terms of a state of cerebral Aβ deposition, the analysis method comprising:
before and after the intervention performed on the AD model mouse,
measuring a level of mouse Aβ1-40 and a level of mouse APP669-711 in a biological sample derived from the AD model mouse, by detection of markers including mouse Aβ1-40 and mouse APP669-711, and
calculating an APP669-71 1/Aβ1-40 ratio which is a ratio of the level of mouse APP669-711 to the level of mouse Aβ1-40; and
comparing the ratio in the AD model mouse before the intervention with the ratio in the AD model mouse after the intervention.

3. An analysis method of judging whether an amount of cerebral Aβ deposition is increased or decreased, the analysis method comprising:
performing, on a same AD model mouse over a period of time, multiple sets of a step of measuring a level of mouse Aβ1-40 and a level of mouse APP669-711 in a biological sample derived from the AD model mouse by detection of markers including mouse Aβ1-40 and mouse APP669-711 and a step of calculating an APP669-711/Aβ1-40 ratio which is a ratio of the level of mouse APP669-711 to the level of mouse Aβ1-40; and
evaluating a change in the ratio in the AD model mouse.

4. An analysis method of screening candidate substances intended for improving a state of cerebral Aβ deposition, the analysis method comprising:
before and after providing an AD model mouse with a candidate substance intended for improving a state of cerebral Aβ deposition,
measuring a level of mouse Aβ1-40 and a level of mouse APP669-711 in a biological sample derived from the AD model mouse, by detection of markers including mouse Aβ1-40 and mouse APP669-711, and
calculating an APP669-71 1/Aβ1-40 ratio which is a ratio of the level of mouse APP669-711 to the level of mouse Aβ1-40; and
comparing the ratio in the AD model mouse before the candidate substance is provided, with the ratio in the AD model mouse after the candidate substance is provided.

5. The analysis method according to any one of claims 1 to 4, wherein the biological sample is blood.

6. The analysis method according to any one of claims 1 to 5, wherein the AD model mouse is an APP/PS1 mouse.

7. A marker enabling judgement of a state of cerebral Aβ deposition and consisting of a combination of mouse Aβ1-40 and mouse APP669-711 in a biological sample derived from an AD model mouse.

8. The marker according to claim 7, wherein the biological sample is blood.

9. The marker according to claim 7, wherein the AD model mouse is an APP/PS1 mouse.
